(19) Europäisches Patentamt
European Patent Office
Office européen des brevets

(11) Veröffentlichungsnummer: **0 036 650**
**A2**

# (12) EUROPÄISCHE PATENTANMELDUNG

(21) Anmeldenummer: **81102128.6**

(22) Anmeldetag: **20.03.81**

(51) Int. Cl.³: **C 07 D 501/36**, A 61 K 31/545
// C07D253/06

(30) Priorität: **25.03.80 CH 2327/80**

(43) Veröffentlichungstag der Anmeldung: **30.09.81**
**Patentblatt 81/39**

(84) Benannte Vertragsstaaten: **AT BE CH DE FR GB IT LI LU NL SE**

(71) Anmelder: **F. HOFFMANN-LA ROCHE & CO.**
**Aktiengesellschaft, CH-4002 Basel (CH)**

(72) Erfinder: **Furlenmeier, André, Dr., Wettsteinallee 119, CH-4058 Basel (CH)**
Erfinder: **Lanz, Paul, Freidorfweg 16, CH-4132 Muttenz (CH)**

(74) Vertreter: **Lederer, Franz, Dr. et al, Patentanwälte Dr. Franz Lederer Reiner F. Meyer Lucile-Grahn-Strasse 22, D-8000 München 80 (DE)**

## (54) Cephalosporinderivate, entsprechende Präparate, die Verwendung dieser Produkte bei der Behandlung von Krankheiten, die Herstellung der Wirkstoffe sowie dabei anfallende Zwischenprodukte.

(57) Cephalosporinderivat der Formel

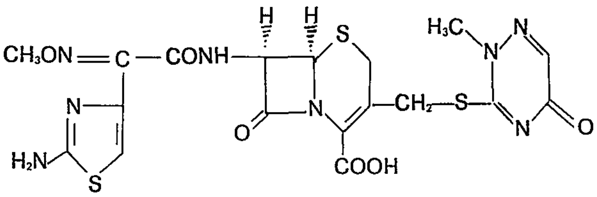

I

sowie leicht hydrolysierbare Ester und Salze dieser Verbindung und Hydrate der Verbindung der Formel I bzw. von deren Estern und Salzen.

Ferner auch entsprechende Präparate, die Verwendung dieser Produkte bei der Behandlung von Krankheiten, die Herstellung der Wirkstoffe sowie dabei anfallende Zwischenprodukte.

EP 0 036 650 A2

F.Hoffmann-La Roche & Co. Aktiengesellschaft, Basel, Schweiz

RAN 4410/144

Cephalosporinderivate, entsprechende Präparate, die Verwendung dieser Produkte bei der Behandlung von Krankheiten, die Herstellung der Wirkstoffe sowie dabei anfallende Zwischenprodukte.

Die vorliegende Erfindung betrifft neue Cephalosporinderivate, und zwar ein Cephalosporinderivat der Formel

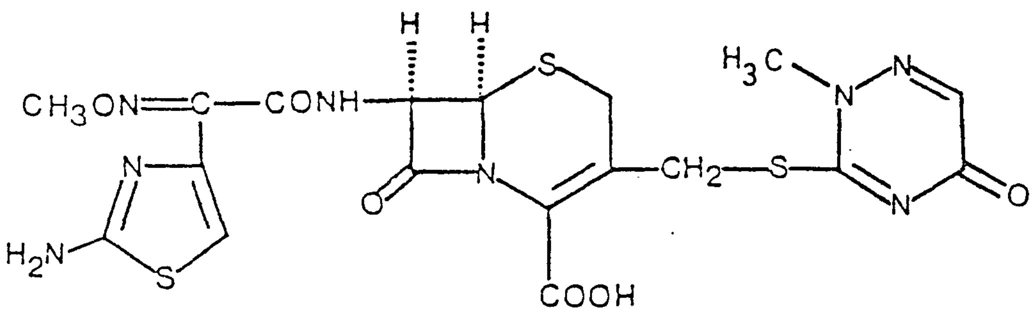

I

sowie leicht hydrolysierbare Ester und Salze dieser Verbindung und Hydrate der Verbindung der Formel I bzw. von deren Estern und Salzen.

Als leicht hydrolysierbare Ester der Verbindung der Formel I sind Verbindungen der Formel I zu verstehen, deren Carboxygruppe in Form einer leicht hydrolysierbaren Estergruppe vorliegt. Beispiele solcher Ester, die her-

Mn/6.1.1981

kömmlicher Art sein können, sind die niederen Alkanoyloxyalkylester, z.B. der Acetoxymethyl-, Pivaloyloxymethyl-, 1-Acetoxyäthyl- und 1-Pivaloyloxyäthylester; die niederen Alkoxycarbonyloxyalkylester, z.B. der Methoxycarbonyloxymethyl-, 1-Aethoxycarbonyloxyäthyl- und 1-Isopropoxycarbonyloxyäthylester; die Lactonylester, z.B. der Phthalidyl- und Thiophthalidylester; die niederen Alkoxymethylester, z.B. der Methoxymethylester; und die niederen Alkanoylaminomethylester, z.B. der Acetamidomethylester. Auch andere Ester, z.B. die Benzyl- und Cyanmethylester, können brauchbar sein.

Beispiele von Salzen der Verbindung der Formel I sind Alkalimetallsalze, wie das Natrium- und Kaliumsalz; das Ammoniumsalz; Erdalkalimetallsalze, wie das Calciumsalz; Salze mit organischen Basen, wie Salze mit Aminen, z.B. Salze mit N-Aethyl-piperidin, Procain, Dibenzylamin, N,N'-Dibenzyläthylendiamin, Alkylaminen oder Dialkylaminen, sowie Salze mit Aminosäuren, wie z.B. Salze mit Arginin oder Lysin.

Die Verbindung der Formel I bildet ebenfalls Additionssalze mit organischen oder anorganischen Säuren. Beispiele solcher Salze sind Hydrohalogenide, beispielsweise Hydrochloride, Hydrobromide, Hydrojodide, sowie andere Mineralsäuresalze, wie Sulfate, Nitrate, Phosphate und dgl., Alkyl- und Monoaryl-sulfonate, wie Aethansulfonate, Toluolsulfonate, Benzolsulfonate und dgl. und auch andere organische Säuresalze, wie Acetate, Tartrate, Maleate, Citrate, Benzoate, Salicylate, Ascorbate und dgl.

Die Verbindung der Formel I (einschliesslich deren Salze und leicht hydrolysierbare Ester) können hydratisiert sein. Die Hydratisierung kann im Zuge des Herstellungsverfahrens erfolgen oder allmählich als Folge hygroskopischer Eigenschaften eines zunächst wasserfreien Produktes auftreten.

Die erfindungsgemässen Produkte können in der syn-isomeren Form

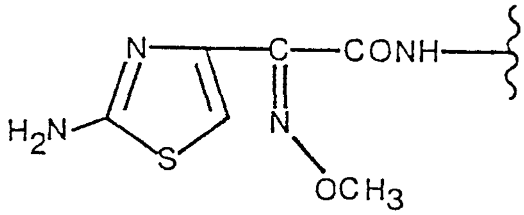

oder in der anti-isomeren Form

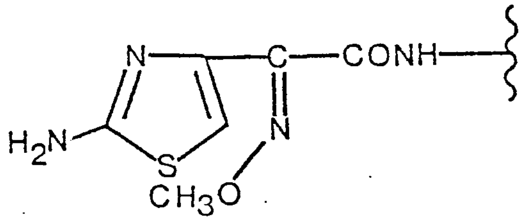

bzw. als Gemische dieser beiden Formen vorliegen. Bevorzugt ist die syn-isomere Form bzw. Gemische, in denen die syn-isomere Form überwiegt.

Bevorzugt ist die Verbindung der Formel I, d.h.

(6R,7R)-7-[2-(2-Amino-4-thiazolyl)-2-[(Z)-methoxyimino]acetamido]-3-[[(2,5-dihydro-2-methyl-5-oxo-as-triazin-3-yl)thio]methyl]-8-oxo-5-thia-1-azabicyclo[4.2.0]oct-2-en-2-carbonsäure,

und deren Salze sowie die entsprechenden Hydrate.

Die obigen Cephalosporinderivate werden erfindungsgemäss dadurch hergestellt dass man

a) in einer Verbindung der allgemeinen Formel

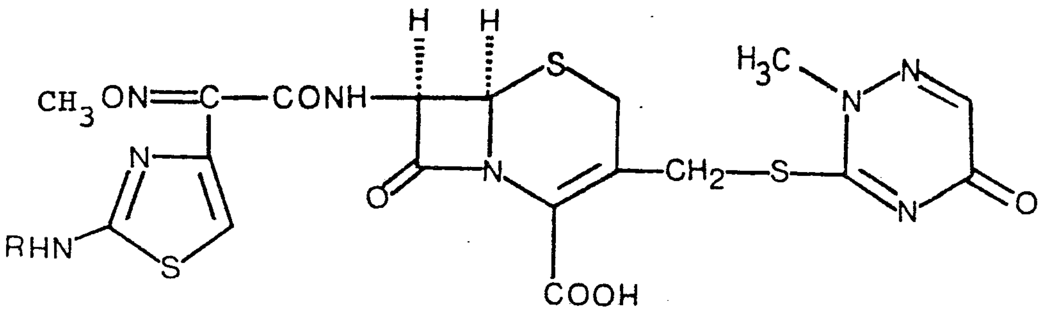

II

in der R eine abspaltbare Schutzgruppe darstellt und die Carboxygruppe in geschützter Form vorliegen kann, die Schutzgruppe R, ggfs. auch eine allenfalls vorhandene Carboxyschutzgruppe, abspaltet, oder dass man

b) ein Halogenid der allgemeinen Formel

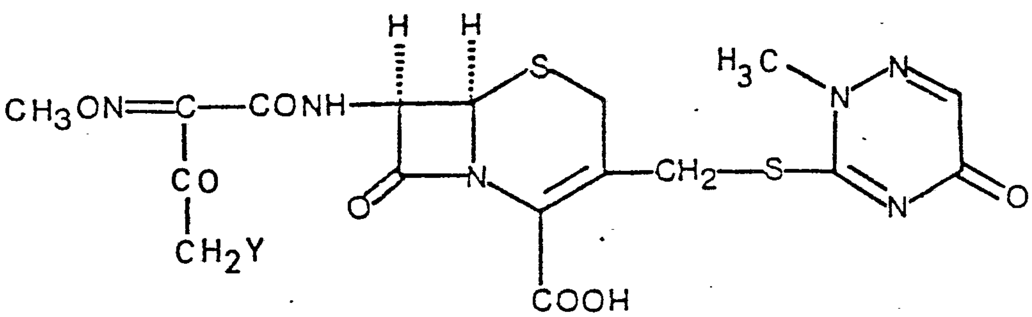

III

in der Y ein Halogenatom darstellt und die Carboxygruppe in geschützter Form vorliegen kann, mit Thioharnstoff umsetzt, oder dass man

c) zur Herstellung eines leicht hydrolysierbaren Esters der Carbonsäure der Formel I diese einer entsprechenden Veresterung unterwirft, oder dass man

d) zur Herstellung von Salzen oder Hydraten der Verbindung der Formel I bzw. Hydraten dieser Salze die Verbindung der Formel I in ein Salz oder Hydrat bzw. ein

0036650

Hydrat dieses Salzes überführt.

Die in den Ausgangsverbindungen der Formeln II und III vorhandene Carboxygruppe kann wahlweise geschützt sein, z.B. durch Veresterung zu einem leicht spaltbaren Ester, wie einem Silylester, z.B. dem Trimethylsilylester. Es kommen auch die oben erläuterten, leicht hydrolysierbaren Ester in Betracht. Die Carboxygruppe kann auch durch Salzbildung mit einer anorganischen oder tertiären organischen Base, wie Triäthylamin, geschützt werden. Mögliche R-Schutzgruppen sind beispielsweise sauer-hydrolytisch abspaltbare Schutzgruppen, wie z.B. t-Butoxycarbonyl oder Trityl, oder auch basisch-hydrolytisch abspaltbare Schutzgruppen, wie z.B. Trifluoracetyl. Bevorzugte R-Schutzgruppen sind Chlor-, Brom- und Jodacetyl, insbesondere Chloracetyl. Letztere Schutzgruppen können durch Behandeln mit Thioharnstoff abgespalten werden.

Die Ausgangsverbindungen der Formel II können z.B. durch N-Acylierung der entsprechenden 7-Aminoverbindung hergestellt werden, und zwar indem man eine Verbindung der Formel

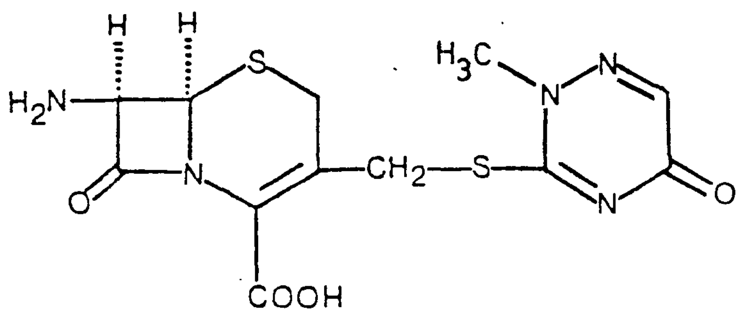

IV

in der die Carboxygruppe und/oder die Aminogruppe in geschützter Form vorliegen kann,

mit einer Säure der allgemeinen Formel

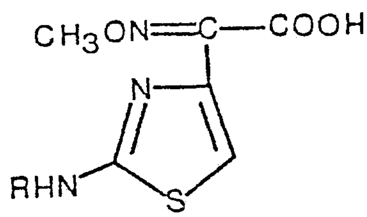

V

in der R die oben gegebene Bedeutung hat, oder mit einem reaktionsfähigen funktionellen Derivat dieser Säure umsetzt und eine allenfalls vorhandene Carboxyschutzgruppe gegebenenfalls abspaltet.

Die in der 7-Aminoverbindung der Formel IV vorhandene Carboxygruppe kann wahlweise geschützt sein, und zwar in der oben für die herzustellende Ausgangsverbindung der Formel II erläuterten Weise. Die Aminogruppe der Verbindung der Formel IV kann z.B. durch eine Silylschutzgruppe, wie Trimethylsilyl, geschützt sein.

Als reaktionsfähige funktionelle Derivate von Säuren der Formel V kommen z.B. Halogenide, d.h. Chloride, Bromide und Fluoride; Azide; Anhydride, insbesondere gemischte Anhydride mit stärkeren Säuren; reaktionsfähige Ester, z.B. N-Hydroxysuccinimidester, und Amide, z.B. Imidazolide, in Betracht.

Die Umsetzung der 7-Aminoverbindung der Formel IV mit der Säure der Formel V oder einem reaktionsfähigen funktionellen Derivat davon kann in an sich bekannter Weise durchgeführt werden. So kann man z.B. eine freie Säure der Formel V mit einem der erwähnten Ester entsprechend Formel IV mittels eines Carbodiimids, wie Dicyclohexylcarbodiimid, in einem inerten Lösungsmittel, wie Aethylacetat,Acetonitril,Dioxan, Chloroform, Methylenchlorid, Benzol oder Dimethylformamid, kondensieren und anschliessend die Estergruppe abspalten. Anstelle von Carbodiimiden lassen sich als Kondensationsmittel auch Oxazoliumsalze, z.B. N-Aethyl-5-phenyl-isoxazolium-3'-sulfonat, verwenden.

Nach einer anderen Ausführungsform setzt man ein Salz einer Säure der Formel IV z.B. ein Trialkylammoniumsalz, wie das Triäthylammoniumsalz, mit einem reaktionsfähigen funktionellen Derivat einer Säure der Formel V wie oben erwähnt in einem inerten Lösungsmittel, z.B. einem der oben genannten, um.

Nach einer weiteren Ausführungsform wird ein Säurehalogenid, vorzugsweise das Chlorid einer Säure der Formel V mit dem Amin der Formel IV umgesetzt. Die Umsetzung erfolgt vorzugsweise in Gegenwart eines säurebindenden Mittels, z.B. in Gegenwart von wässrigem Alkali, vorzugsweise Natronlauge, oder auch in Gegenwart eines Alkalimetallcarbonats, wie Kaliumcarbonat, oder in Gegenwart eines nieder-alkylierten Amins, wie Triäthylamin. Als Lösungsmittel wird vorzugsweise Wasser verwendet, ggfs. im Gemisch mit einem inerten organischen Lösungsmittel, wie Tetrahydrofuran oder Dioxan. Es kann auch in einem aprotischen organischen Lösungsmittel, wie z.B. Dimethylformamid, Dimethylsulfoxid oder Hexamethylphosphorsäuretriamid, gearbeitet werden. Bei Verwendung von silylierten Ausgangsverbindungen der Formel IV wird in wasserfreiem Medium gearbeitet.

Die Umsetzung der 7-Aminoverbindung der Formel IV mit der Säure der Formel V oder einem reaktionsfähigen funktionellen Derivat davon kann zweckmässig bei Temperaturen zwischen etwa -40°C und Zimmertemperatur, beispielsweise bei etwa 0-10°C, erfolgen.

Ausgangsverbindungen der Formel II, worin R nicht monohalogeniert ist, wie in Brom-, Chlor- und Jodacetyl, können auch durch Thiolierung hergestellt werden, und zwar indem man eine Verbindung der allgemeinen Formel

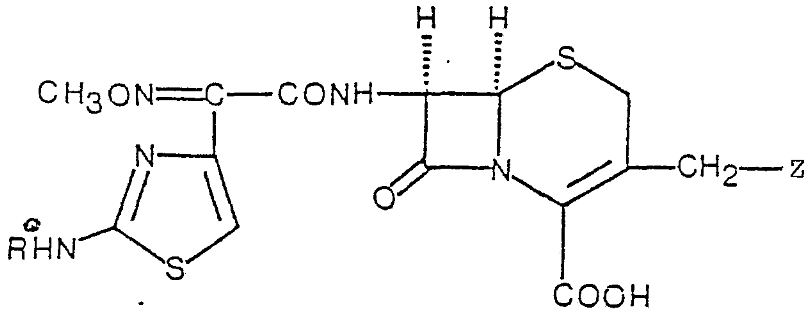

VI

in der R° wie R ist, jedoch nicht monohalogeniert sein kann, Z eine austretende Gruppe darstellt und die Carboxygruppe durch Salzbildung mit einer anorganischen oder tertiären organischen Base geschützt sein kann,

mit einem Thiol der Formel

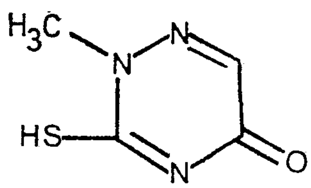

VII

umsetzt und eine allenfalls vorhandene Carboxyschutzgruppe gegebenenfalls abspaltet.

Als austretende Gruppe Z einer Verbindung der Formel VI kommen beispielsweise Halogene, z.B. Chlor, Brom oder Jod, Acyloxyreste, z.B. niedere Alkanoyloxyreste, wie Acetoxy, niedere Alkyl- oder Arylsulfonyloxyreste, wie Mesyloxy oder Tosyloxy, oder der Azidorest in Frage.

Die Umsetzung der Verbindung der Formel VI mit dem Thiol der Formel VII kann in an sich bekannter Weise, z.B. bei einer Temperatur zwischen etwa 40 und 80°C, zweckmässig bei etwa 60°C,

vorzugsweise in Wasser oder in einer Pufferlösung mit einem pH von etwa 6 bis 7, vorzugsweise 6,5, durchgeführt werden.

Die Carboxygruppe der erhaltenen Verbindungen II kann erwünschtenfalls anschliessend geschützt werden, z.B. durch Salzbildung oder Veresterung.

Das Thiol der Formel VII steht in tautomerem Gleichgewicht mit dem entsprechenden Thion. Dessen Herstellung wird in Beispiel 1 beschrieben.

Gemäss Verfahrensvariante a) des erfindungsgemässen Verfahrens wird die Aminoschutzgruppe R einer Ausgangsverbindung der Formel II abgespalten. Durch saure Hydrolyse abspaltbare Schutzgruppen werden vorzugsweise mit Hilfe einer niederen Alkancarbonsäure, die ggfs. halogeniert sein kann, entfernt. Insbesondere verwendet man Ameisensäure oder Trifluoressigsäure. Die Temperatur ist in der Regel Raumtemperatur, obwohl auch leicht erhöhte bzw. leicht erniedrigte Temperatur angewendet werden kann, z.B. im Bereiche von etwa $0^{o}C$ bis $+40^{o}C$. Alkalisch abspaltbare Schutzgruppen werden im allgemeinen mit verdünnter wässriger Lauge bei $0^{o}C$ bis $30^{o}C$ hydrolysiert. Die Chloracetyl-, Bromacetyl- und Jodacetyl-Schutzgruppen können mittels Thioharnstoff in saurem, neutralem oder alkalischem Milieu bei etwa $0-30^{o}C$ abgespalten werden. Hydrogenolytische Abspaltung (z.B. Abspaltung von Benzyl) ist hier ungeeignet, da bei der Hydrogenolyse die Oximfunktion zur Aminogruppe reduziert wird.

Nach Durchführung der Verfahrensvariante a) kann, falls erwünscht, eine allenfalls im Reaktionsprodukt vorhandene Carboxyschutzgruppe abgespalten werden. Wenn die Schutzgruppe eine Silylgruppe darstellt (Silylester), kann diese Gruppe besonders leicht durch Behandeln des Umsetzungsproduktes mit Wasser abgespalten werden. Niedere Alkanoyloxyalkyl-, Alkoxycarbonyloxyalkyl-, Lacto-

nyl-, Alkoxymethyl- und Alkanoylaminomethylester werden vorzugsweise enzymatisch mit Hilfe einer geeigneten Esterase (bei etwa 20-40°C) abgespalten. Wenn die Carboxygruppe durch Salzbildung (z.B. mit Triäthylamin) geschützt ist, so kann die Abspaltung dieser salzbildenden Schutzgruppe durch Behandlung mit Säure erfolgen. Als Säure kann hierbei z.B. Salzsäure, Schwefelsäure, Phosphorsäure oder Citronensäure verwendet werden.

Die Carboxyschutzgruppe kann in der gleichen Weise wie soeben beschrieben auch vor der Abspaltung der Schutzgruppe R abgespalten werden.

Das erfindungsgemäss eingesetzte Halogenid der Formel III kann z.B. durch Umsetzung einer Verbindung der Formel IV mit einer halogenierten Carbonsäure der allgemeinen Formel

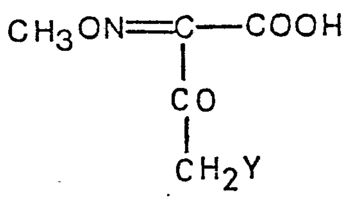

VIII

in der Y ein Halogenatom darstellt, oder mit einem reaktionsfähigen Derivat dieser Verbindung hergestellt werden. Die halogenierte Carbonsäure der Formel VIII wird entweder in freier Form eingesetzt in Gegenwart eines Kondensationsmittel, z.B. eines N,N'-disubstituierten Carbodiimides, wie N,N'-dicyclohexylcarbodiimid, oder einer Azolidverbindung, wie N,N'-Carbonyldiimidazol oder N,N'-Thionyldiimidazol oder auch in Form eines Säurehalogenids, wie des Säurechlorids oder -bromids, in Form eines Säureanhydrids, wie eines Säureanhydrids mit einem Kohlensäuremonoester, z.B. mit Monomethyl- oder Monoisopropylcarbonat, oder in Form eines aktivierten Esters, wie des p-Nitrophenylester, 2,4-Dinitrophenylesters, N-Hydroxysuccinimidesters oder N-Hydroxyphthalimidesters. Die Reaktion erfolgt im allgemeinen in

einem inerten organischen Lösungsmittel, z.B. in einem halogenierten Kohlenwasserstoff, wie Chloroform, Dichlormethan, Tetrachlorkohlenstoff, in einem Aether, z.B. Tetrahydrofuran, Dioxan, in Dimethylformamid, Dimethylacetamid, Wasser oder Mischungen davon. Die Reaktionstemperatur liegt vornehmlich im Bereich von etwa -50 bis +40°C, vorzugsweise bei etwa -10 bis +10°C.

Die erfindungsgemässe Umsetzung des Halogenids der Formel III mit Thioharnstoff (Variante b) des erfindungsgemässen Verfahrens) verläuft vorzugsweise in einem inerten Lösungsmittel, wie z.B. in einem niederen Alkanol, z.B. Aethanol, in einem niederen Keton, wie Aceton, in einem Aether, wie Tetrahydrofuran, Dioxan, in Dimethylformamid, Dimethylacetamid, in Wasser oder in Mischungen davon. Die Reaktionstemperatur liegt im allgemeinen im Bereich von etwa 0°C bis 60°C, vorzugsweise bei Zimmertemperatur. Als Halogenid der Formel III kann das Chlorid, Bromid, Fluorid oder Jodid eingesetzt werden, bevorzugt verwendet man das Chlorid oder das Bromid. Es kann die freie Säure der Formel III eingesetzt werden, wahlweise aber auch ein Salz davon, wobei die gleichen Salze wie die oben erläuterten Salze der Verbindung der Formel I in Betracht kommen.

Zur Herstellung der leicht hydrolysierbaren Ester der Carbonsäure der Formel I gemäss Variante c) wird diese vorzugsweise mit dem entsprechenden, die Estergruppe enthaltenden Halogenid, bevorzugt mit dem Jodid, umgesetzt. Die Reaktion kann mit Hilfe einer Base, z.B. einem Alkalimetallhydroxid oder -carbonat, oder einem organischen Amin, wie Triäthylamin, beschleunigt werden. Die Veresterungsreaktion wird vorzugsweise in einem inerten organischen Lösungsmittel durchgeführt, wie Dimethylacetamid, Hexamethylphosphorsäuretriamid, Dimethylsulfoxid oder, vorzugsweise, Dimethylformamid. Die Temperatur liegt vorzugsweise im Bereiche von etwa 0-40°C.

Die Herstellung der Salze und Hydrate der Verbindung der Formel I bzw. der Hydrate dieser Salze kann in an sich bekannter Weise erfolgen, z.B. durch Umsetzung der Carbonsäure der Formel I mit einer äquivalenten Menge der gewünschten Base, zweckmässig in einem Lösungsmittel, wie Wasser oder in einem organischen Lösungsmittel, wie Aethanol, Methanol, Aceton und anderen mehr. Die Temperatur der Salzbildung ist nicht kritisch. Sie liegt im allgemeinen bei Raumtemperatur, kann aber auch leicht darüber oder darunter, etwa im Bereiche von 0°C bis +50°C, sein.

Die Herstellung der Hydrate erfolgt zumeist automatisch im Zuge des Herstellungsverfahrens oder als Folge hygroskopischer Eigenschaften eines zunächst wasserfreien Produktes. Zur gezielten Herstellung eines Hydrats kann ein ganz oder teilweise wasserfreies Produkt (Carbonsäure der Formel I bzw. Ester, Aether oder Salz davon) einer feuchten Atmosphäre, z.B. bei etwa +10°C bis +40°C, ausgesetzt werden.

Die oben verwendete 7-Aminoverbindung Z der Formel IV kann ausgehend von einer Verbindung der Formel

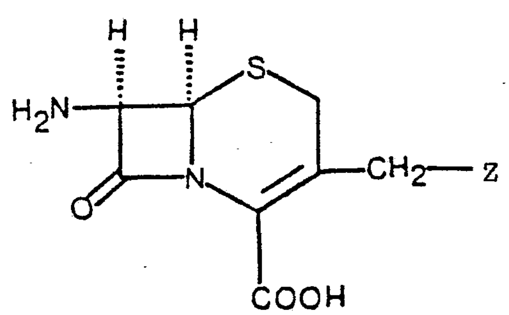

IX

in der Z eine austretende Gruppe darstellt und die Carboxygruppe durch Salzbildung mit einer anorganischen oder tertiären organischen Base geschützt sein kann,

mit dem Thiol der Formel VII hergestellt werden. Die Umsetzung kann unter den gleichen Bedingungen wie denjenigen erfolgen, wie sie für die Umsetzung der Ausgangsverbindungen VI und VII beschrieben wurden. Andererseits können

die Verbindungen der Formel VI ausgehend von einer Verbindung der Formel IX und einer Säure der Formel V oder einem reaktionsfähigen funktionellen Derivat davon unter den gleichen Bedingungen hergestellt werden, wie sie für die Umsetzung der Verbindungen der Formeln IV und V beschrieben wurden.

Die Carboxygruppe und/oder die Aminogruppe der erhaltenen Verbindung IV können erwünschtenfalls anschliessend geschützt werden, z.B. durch Veresterung oder Salzbildung der Carboxygruppe oder durch Silylierung der Aminogruppe.

Ein allenfalls erhaltenes syn/anti-Gemisch einer Verbindung der Formel I kann in die entsprechende syn- und anti-Form in üblicher Weise aufgetrennt werden, beispielsweise durch Umkristallisation oder durch chromatographische Methoden unter Verwendung eines geeigneten Lösungsmittels bzw. Lösungsmittelgemisches.

Die Verbindungen der Formeln I und II sowie die entsprechenden leicht hydrolysierbaren Ester und Salze bzw. die Hydrate dieser Produkte sind antibiotisch, insbesondere bakterizid wirksam. Sie besitzen ein breites Wirkungsspektrum gegen Gram-positive Bakterien, z.B. Staphylokokken, und gegen Gram-negative Bakterien, wie z.B. Haemophilus influenzae, Neisseria gonorrhoeae, sowie gegen verschiedene ß-Lactamase-bildende Gram-negative Erreger, wie Escherichia coli, Serratia marcescens, Pseudomonas aeruginosa, Proteus mirabilis, Proteus vulgaris.

Die Verbindungen der Formeln I und II sowie die entsprechenden leicht hydrolysierbaren Ester und Salze bzw. die Hydrate dieser Produkte können zur Behandlung und Prophylaxe von Infektionskrankheiten verwendet werden. Für den Erwachsenen kommt eine Tagesdosis von etwa 0,1 g bis etwa 2 g in Betracht. Die parenterale Verabreichung der erfindungsgemässen Verbindungen ist besonders bevorzugt.

Zum Nachweis der antimikrobiellen Wirksamkeit der erwähnten Produkte wurde das (6R,7R)-7-[2-(2-Amino-5-thiazolyl)-2-[(Z)-methoxyimino]acetamido]-3-[[(2,5-dihydro-2-methyl-5-oxo-as-triazin-3-yl)thio]methyl]-8-oxo-5-thia-1-azabicyclo[4.2.0]oct-2-en-2-carbonsäure-Natriumsalz auf seine Aktivität in vitro gegen verschiedene Krankheitserreger getestet. Nachstehend sind die entsprechenden Mindesthemmkonzentrationen (MIC; µg/ml) zusammengestellt:

| Krankheitserreger | MIC µg/ml |
|---|---|
| Escherichia coli*) | 0,04 |
| Serratia marcescens*) | 0,16 |
| Enterobacter cloacae*) | 2,5 |
| Proteus mirabilis*) | 0,04 |
| Proteus vulgaris*) | 0,02 |
| Pseudomonas aeruginosa | |
| Stamm 1*) | 40 |
| Stamm 2*) | 40 |
| Haemophilus influenzae | 0,02 |
| Neisseria gonorrhoeae | 0,02 |
| Staphylococcus aureus | 2,5 |

*) ß-Lactamase bildende Stämme

Toxizität

| Prüfsubstanz | |
|---|---|
| $LD_{100}$, mg/kg i.v. | 1000 |
| s.c. | >5000 |
| p.o. | >5000 |

Die erfindungsgemässen Produkte können als Heilmittel z.B. in Form pharmazeutischer Präparate Verwendung finden, welche sie oder ihre Salze in Mischung mit einem für die enterale oder parenterale Applikation geeigneten pharmazeutischen, organischen oder anorganischen inerten Trägermaterial, wie z.B. Wasser, Gelatine, Gummi arabicum, Milchzucker, Stärke, Magnesiumstearat, Talk, pflanzliche Oele, Polyalkylenglykole, Vaseline, usw. enthalten. Die pharmazeutischen Präparate können in fester Form, z.B. als Tabletten, Dragées, Suppositorien, Kapseln; oder in flüssiger Form, z.B. als Lösungen, Suspensionen oder Emulsionen, vorliegen. Gegebenenfalls sind sie sterilisiert und bzw. oder enthalten Hilfsstoffe, wie Konservierungs-, Stabilisierungs-, Netz- oder Emulgiermittel, Salze zur Veränderung des osmotischen Druckes, Anaestetica oder Puffer. Sie können auch noch andere therapeutisch wertvolle Stoffe enthalten. Die Verbindung der Formel I und ihre Salze bzw. Hydrate kommen vorzugsweise für die parenterale Applikation in Betracht und werden zu diesem Zweck bevorzugt als Lyophilisate oder Trockenpulver zur Verdünnung mit üblichen Agenzien, wie Wasser oder isotonische Kochsalzlösung, zubereitet. Die leicht hydrolysierbaren Ester der Verbindung der Formel I und ihre Salze bzw. Hydrate kommen auch für die enterale Verabreichung in Betracht.

In den nachstehenden Ausführungsbeispielen sind alle Temperaturen in $^{o}$Celsius angegeben.

## Beispiel 1

(6R,7R)-7-[2-(2-Amino-4-thiazolyl)-2-[(Z)-methoxyimino]acetamido]-3-[[(2,5-dihydro-2-methyl-5-oxo-as-triazin-3-yl)thio]methyl]-8-oxo-5-thia-1-azabicyclo[4.2.0]oct-2-en-2-carbonsäure-Natriumsalz

61 g (6R,7R)-7-[2-(Chloracetamido-4-thiazolyl)-2-[(Z)-methoxyimino]-acetamido]-3-[[(2,5-dihydro-2-methyl-5-oxo-as-triazin-3-yl)thio]methyl]-8-oxo-5-thia-1-azabicyclo[4.2.0]oct-2-en-2-carbonsäure werden in 1,5 l Wasser mit 30,4 g Thioharnstoff versetzt und unter Stickstoffbegasung und Rühren mit 1N Natronlauge via Autotitrator während 20 Stunden bei einem pH-Wert von 7,0 gehalten. Durch Eintropfen von 1N HCl wird unter Rühren der pH-Wert auf 3,75 gestellt. Der ausgefallene Niederschlag wird abgesaugt und verworfen. Nun wird das Filtrat unter Rühren mit 1N Salzsäure auf einen pH-Wert von 3,0 gestellt, der Niederschlag abgesaugt, mit Wasser, Aethanol und Petroläther gewaschen und getrocknet. Das erhaltene Rohprodukt wird in 90 ml Aceton/Wasser (1:1) suspendiert, mit 45 ml einer 2N Lösung von Natrium-2-aethylcaproat in Aethylacetat versetzt, gerührt bis zur vollständigen Lösung und danach unter Eintropfen in 800 ml Aceton unter Rühren ausgefällt. Das Natriumsalz wird abgesaugt, mit Aceton, Aether und Petroläther gewaschen und getrocknet. Zur weiteren Reinigung wird die Substanz in 800 ml Methanol unter Rühren gelöst und von wenig ungelöster Substanz abfiltriert. Das Filtrat wird mit Aktivkohle behandelt, filtriert, im Vakuum auf ca. 400 ml eingedampft und von wenig ausgefallener Substanz abfiltriert. Das Filtrat wird unter Eintropfen von 1,6 l Aethylacetat ausgefällt, abgesaugt, mit Aethylacetat, Aether und Petroläther gewaschen und getrocknet. Man erhält (6R,7R)-7-[2-(2-Amino-4-thiazolyl)-2-[(Z)-methoxyimino]acetamido]-3-[[(2,5-dihydro-2-methyl-5-oxo-as-triazin-3-yl)thio]methyl]-8-oxo-5-thia-1-azabicyclo[4.2.0]oct-2-en-carbonsäure-Natriumsalz; Smp. = ab 192° langsame Zers. $[\alpha]_D^{25}$ = -135° (c = 1 in Wasser).

Die als Ausgangssubstanz verwendete (6R,7R)-7-[2-(Chloracetamido-4-thiazolyl)-2-[(Z)-methoxyimino]-acetamido]-3-[[(2,5-dihydro-2-methyl-5-oxo-as-triazin-3-yl)thio]methyl]-8-oxo-5-thia-1-azabicyclo[4.2.0]oct-2-en-2-carbonsäure kann wie folgt hergestellt werden:

<u>2-Methyl-5-oxo-3-thioxo-2,3,4,5-as-triazin</u>

In eine Lösung von 80,8 g Glyoxylsäure-Hydrat in 450 ml Dimethylformamid werden portionenweise 84 g 2-Methylthiosemicarbazid eingetragen. Die Suspension wird während 45 Minuten bei 80° gerührt. Das Reaktionsgemisch wird abgekühlt, mit 2,5 l Wasser versetzt und die Suspension 1 Stunde bei 0° gerührt. Nach dem Absaugen, Waschen mit Wasser und Trocknen erhält man das 2-Methyl-thiosemicarbazon der Glyoxylsäure, Smp. 204-205° (Zers.). Diese Substanz wird unter Rühren in eine Lösung von 74 g Natriumcarbonat in 700 ml Wasser eingetragen und danach während 3 Stunden unter Rühren auf 95-98° erhitzt. Die Lösung wird auf 5° abgekühlt und unter Rühren und Eintropfen von konz. Salzsäure auf einen pH-Wert von 2 gestellt. Nach dem Abnutschen, Auswaschen mit Eiswasser und Trocknen erhält man 2-Methyl-5-oxo-3-thioxo-2,3,4,5-as-triazin, Smp. 221-222°.

<u>(6R,7R)-7-Amino-3-[[(2,5-dihydro-2-methyl-5-oxo-as-triazin-3-yl)thio]methyl]-8-oxo-5-thia-1-azabicyclo[4.2.0]oct-2-en-2-carbonsäure</u>

27,2 g 7-Amino-cephalosporansäure werden in 200 ml Wasser unter Rühren zuerst portionenweise mit 23,1 g Natriumhydrogencarbonat und danach mit 21,5 g 2-Methyl-5-oxo-3-thioxo-2,3,4,5-as-triazin versetzt. Anschliessend wird unter Stickstoffbegasung während 5 Stunden auf 60°

erwärmt. Die Lösung wird auf 5° abgekühlt, mit konz. Salzsäure auf einen pH-Wert von 3,5 gestellt, die ausgefallene Substanz abfiltriert, mit Wasser gewaschen und zweimal mit je 230 ml Methanol verrührt, abfiltriert, mit Methanol und Aether gewaschen und im Vakuum getrocknet. Man erhält (6R,7R)-7-Amino-3-[[(2,5-dihydro-2-methyl-5-oxo-as-triazin-3-yl)thio]methyl]-8-oxo-5-thia-1-azabicyclo[4.2.0]oct-2-en-2-carbonsäure, Smp.: ab 195° Zers., welche ohne weitere Reinigung weiter umgesetzt wird.

(6R,7R)-7-[2-(Chloracetamido-4-thiazolyl)-2-[(Z)-methoxyimino]acetamido]-3-[[(2,5-dihydro-2-methyl-5-oxo-as-triazin-3-yl)thio]methyl]-8-oxo-5-thia-1-azabicyclo[4.2.0]oct-2-en-2-carbonsäure

In eine -20° kalte Suspension von 34,5 g Phosphorpentachlorid in 500 ml Dichlormethan tropft man 63 ml N,N'-Dimethylformamid, rührt 10 Minuten bei -15°, kühlt danach auf -25°, versetzt mit 46 g 2-(2-Chloracetamido-4-thiazolyl)-2-methoxyimino-essigsäure (syn-Form) und rührt 45 Minuten bei -15°. Die Lösung wird auf -30° gekühlt, mit 50 g Eis vermischt, wobei die Temperatur auf -15° ansteigt. Man rührt 10 Minuten bei -15° und trennt dann die Dichlormethanlösung ab. Sie wird auf -15° gekühlt und sofort für die nachfolgende Acylierung eingesetzt.

Unterdessen werden 61 g (6R,7R)-7-Amino-3-[[2,5-dihydro-2-methyl-5-oxo-as-triazin-3-yl)thio]methyl]-8-oxo-5-thia-1-azabicyclo[4.2.0]oct-2-en-2-carbonsäure in 800 ml Wasser via Autotitrator durch Eintropfen von 2N Natronlauge unter Rühren bei einem pH-Wert von 7,8 gelöst und auf 0° abgekühlt. Bei dieser Temperatur wird innerhalb 45 Minuten die obige -15° kalte Dichlormethanlösung des Säurechlorids zugetropft und durch gleichzeitiges Eintropfen von 2N Natronlauge via Autotitrator ein pH-Wert von 7,8-8,0 eingehalten. Man rührt anschliessend noch während 30 Minuten bei 5° und 1 Stunde bei 20°. Die Lösung wird mit 500 ml

n-Butanol und 500 ml Dichlormethan versetzt, wonach man mit Citronensäure einen pH-Wert von 7 einstellt, 5 Minuten bei diesem pH-Wert rührt und anschliessend im Vakuum filtriert. Die organische Phase wird abgetrennt, 3 mal mit je 200 ml Wasser gewaschen, mit Aktivkohle behandelt, abfiltriert und im Vakuum bei 55° auf ca. 200 ml eingedampft. Die erhaltene Suspension wird auf 20° abgekühlt und im Vakuum filtriert. Man erhält eine 1. Fraktion fester (6R,7R)-7-[2-(Chloracetamido-4-thiazolyl)-2-[(Z)-methoxyimino]-acetamido]-3-[[(2,5-dihydro-2-methyl-5-oxo-as-triazin-3-yl)thio]methyl]-8-oxo-5-thia-1-azabicyclo[4.2.0]oct-2-en-2-carbonsäure. Die Mutterlauge wird im Vakuum auf 100 ml eingedampft, mit 100 ml Aether vermischt und ergibt nach dem Absaugen eine 2. Fraktion der erwähnten Säure. Beide Fraktionen werden gemeinsam in 2 l Aceton unter Rühren innerhalb 15 Minuten gelöst und vom Ungelösten abfiltriert. Zum Filtrat gibt man 300 ml Butylacetat und destilliert das Aceton im Vakuum so lange ab, bis die Substanz kristallisiert. Man erhält (6R,7R)-7-[2-(Chloracetamido-4-thiazolyl)-2-[(Z)-methoxyimino]-acetamido]-3-[[(2,5-dihydro-2-methyl-5-oxo-as-triazin-3-yl)thio]methyl]-8-oxo-5-thia-1-azabicyclo[4.2.0]oct-2-en-2-carbonsäure, Smp. ab 173° Zers., $[\alpha]_D^{25}$ = -238,7° (c = 1 in Dimethylformamid). Das Produkt ist gemäss Dünnschichtchromatographie im System n-Propanol/ Essigsäure/Wasser 55/15/30 einheitlich und wird ohne weitere Reinigung weiter umgesetzt.

## Beispiel 2

Methylen(6R,7R)-7-[2-(2-amino-4-thiazolyl)-2-[(Z)-methoxyimino]acetamido]-3-[[(2,5-dihydro-2-methyl-5-oxo-as-triazin-3-yl)thio]methyl]-8-oxo-5-thia-1-azabicyclo[4.2.0]oct-2-en-2-carboxylat-pivalat

8 g (6R,7R)-7-[2-(2-Amino-4-thiazolyl)-2-[(Z)-methoxyimino]acetamido]-3-[[(2,5-dihydro-2-methyl-5-oxo-as-triazin-3-yl)thio]methyl]-8-oxo-5-thia-1-azabicyclo[4.2.0]oct-2-en-2-carbonsäure-Natriumsalz werden in 80 ml Dimethylformamid gelöst, auf 0° gekühlt, mit 6 g Pivaloyloxymethyljodid versetzt und 30 Minuten bei 0° gerührt. Die Lösung wird in Eiswasser ausgefällt, der Niederschlag im Vakuum abfiltriert, in Aethylacetat unter Zusatz von etwas Methanol gelöst, mit Natriumhydrogencarbonatlösung und Natriumchloridlösung gewaschen, mit Magnesiumsulfat getrocknet und im Vakuum bis zur beginnenden Kristallisation eingedampft. Durch Zusatz eines Gemisches von Aether-Petroläther 1:1 wird die Kristallisation vervollständigt. Die Substanz wird abfiltriert und anschliessend über Kieselgel mit Benzol/Methanol 4:1 chromatographiert. Die einheitlichen Fraktionen werden vereinigt, im Vakuum eingedampft und schliesslich aus Chloroform/Aether kristallisiert. Man erhält Methylen(6R,7R)-7-[2-(2-amino-4-thiazolyl)-2-[(Z)-methoxyimino]acetamido]-3-[[(2,5-dihydro-2-methyl-5-oxo-as-triazin-3-yl)thio]methyl]-8-oxo-5-thia-1-azabicyclo[4.2.0]oct-2-en-2-carboxylat-pivalat. Smp. = 149° Zers., $[\alpha]_D^{25}$ = -173° (c = 1 in Dimethylformamid).

## Beispiel 3

Herstellung von Trockenampullen für die Intramuskuläre Verabreichung:

Es wird in üblicher Weise ein Lyophilisat von 1 g des Wirkstoffes hergestellt und in eine Ampulle abgefüllt. Vor der Verabreichung wird das Lyophilisat mit 2,5 ml einer 2%igen wässrigen Lidocainhydrochlorid-Lösung versetzt.

# Patentansprüche

1. Cephalosporinderivat der Formel

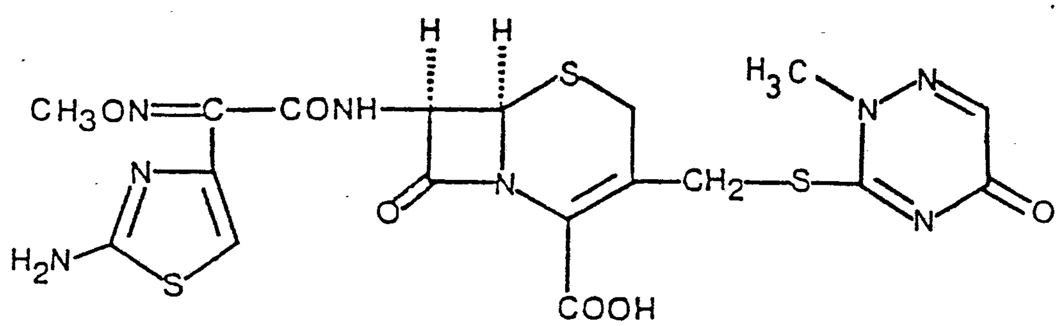

I

sowie leicht hydrolysierbare Ester und Salze dieser Verbindung und Hydrate der Verbindung der Formel I bzw. von deren Estern und Salzen.

2. (6R,7R)-7-[2-(2-Amino-4-thiazolyl)-2-[(Z)-methoxyimino]acetamido]-3-[[(2,5-dihydro-2-methyl-5-oxo-as-triazin-3-yl)thio]methyl]-8-oxo-5-thia-1-azabicyclo[4.2.0]oct-2-en-2-carbonsäure sowie Salze dieser Verbindung und Hydrate dieser Verbindung bzw. Salze.

3. Pivaloyloxymethylester der in Anspruch 1 oder 2 definierten Carbonsäure .

4. Cephalosporinderivate der allgemeinen Formel

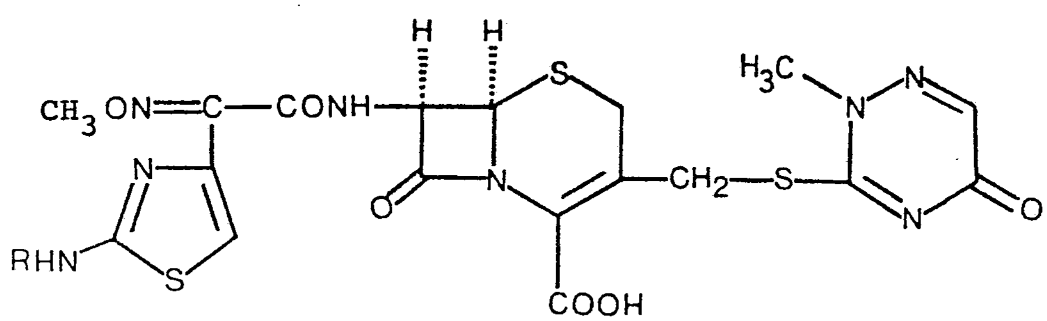

II

in der R eine abspaltbare Schutzgruppe darstellt und die Carboxygruppe in geschützter Form vorliegen kann.

5. Verbindungen der allgemeinen Formel

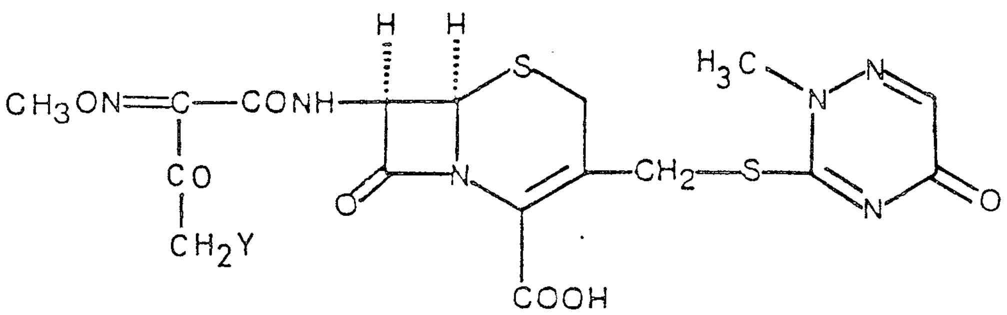

III

in der Y ein Halogenatom darstellt und die Carboxygruppe in geschützter Form vorliegen kann.

6.Eine Verbindung der Formel

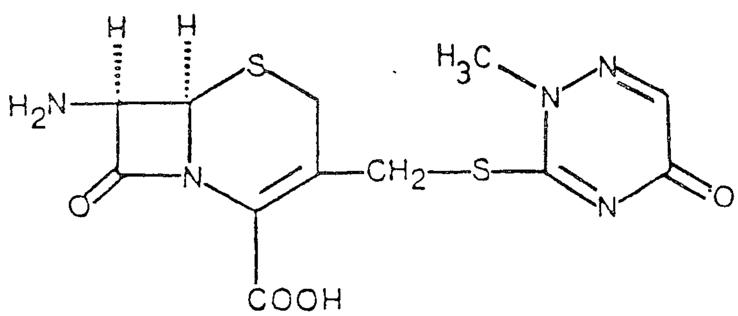

IV

in der die Carboxygruppe und/oder die Aminogruppe in geschützter Form vorliegen kann.

7. Verbindungen gemäss einem der Ansprüche 1-3 als pharmazeutische Wirkstoffe.

8. Verbindungen gemäss einem der Ansprüche 1-3 als pharmazeutische Wirkstoffe zur Behandlung und Prophylaxe von Infektionskrankheiten.

9. Pharmazeutische Präparate, gekennzeichnet durch einen Gehalt an einer Verbindung gemäss einem der Ansprüche 1-3.

10. Pharmazeutische Präparate zur Behandlung und Prophylaxe von Infektionskrankheiten, gekennzeichnet durch einen Gehalt an einer Verbindung gemäss einem der Ansprüche 1-3.

11. Verfahren zur Herstellung von Verbindungen gemäss einem der Ansprüche 1-3, dadurch gekennzeichnet, dass man

a) in einer Verbindung der allgemeinen Formel

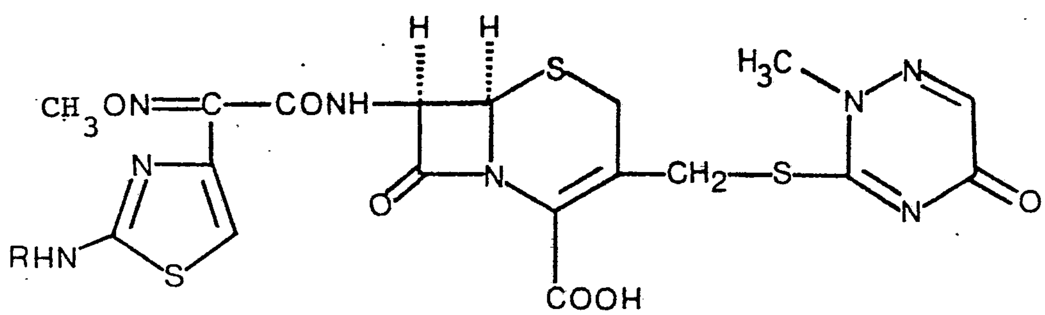

II

in der R eine abspaltbare Schutzgruppe darstellt und die Carboxygruppe in geschützter Form vorliegen kann, die Schutzgruppe R, ggfs. auch eine allenfalls vorhandene Carboxyschutzgruppe, abspaltet, oder dass man

b) ein Halogenid der allgemeinen Formel

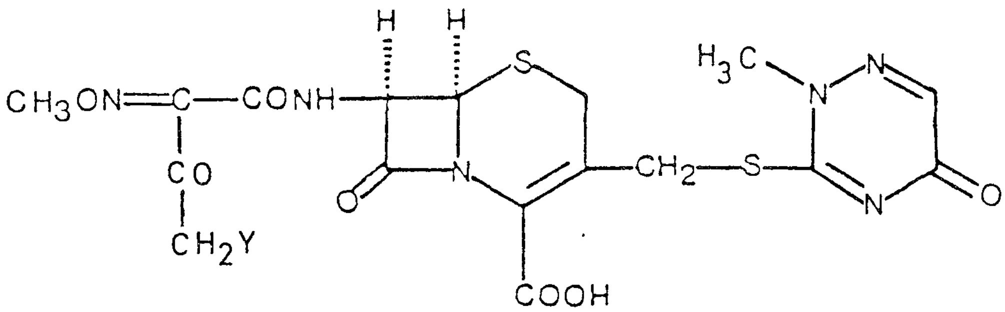

III

in der Y ein Halogenatom darstellt und die Carboxygruppe in geschützter Form vorliegen kann, mit Thioharnstoff umsetzt, oder dass man

c) zur Herstellung eines leicht hydrolysierbaren Esters der Carbonsäure der Formel I diese einer entsprechenden Veresterung unterwirft, oder dass man

d) zur Herstellung von Salzen oder Hydraten der Verbindung der Formel I bzw. Hydraten dieser Salze die Verbindung der Formel I in ein Salz oder Hydrat bzw. ein Hydrat dieses Salzes überführt.

12. Verfahren nach Anspruch 11, dadurch gekennzeichnet, dass man eine Ausgangsverbindung der Formel II oder I einsetzt.

13. Verwendung von Verbindungen gemäss einem der Ansprüche 1-3 bei der Behandlung bzw. Prophylaxe von Krankheiten.

14. Verwendung von Verbindungen gemäss einem der Ansprüche 1-3 bei der Behandlung bzw. Prophylaxe von Infektionskrankheiten.

***

## Patentansprüche für Oesterreich

1. Verfahren zur Herstellung eines Cephalosporinderivats der Formel

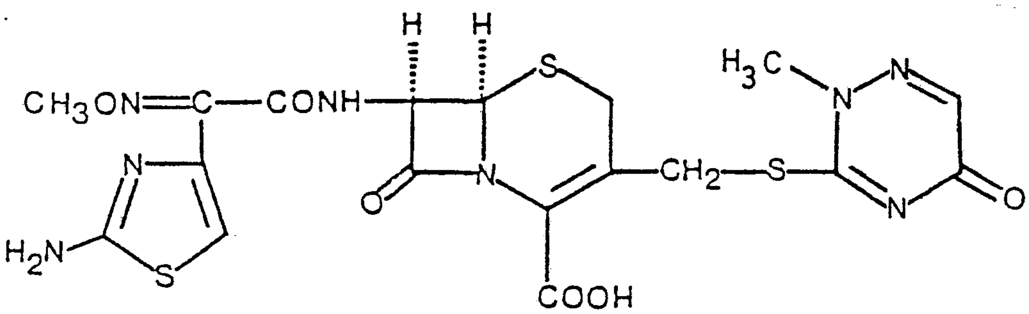

I

sowie leicht hydrolysierbarer Ester und Salze dieser Verbindung und Hydrate der Verbindung der Formel I bzw. von deren Estern und Salzen, dadurch gekennzeichnet, dass man

a) in einer Verbindung der allgemeinen Formel

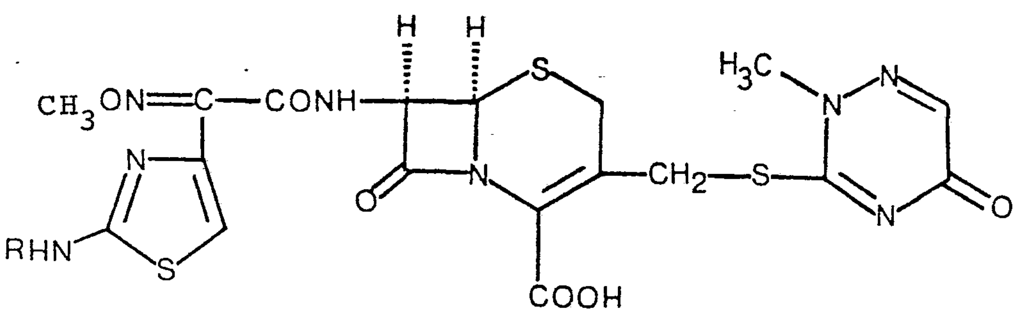

II

in der R eine abspaltbare Schutzgruppe darstellt und die Carboxygruppe in geschützter Form vorliegen kann, die Schutzgruppe R, ggfs. auch eine allenfalls vorhandene Carboxyschutzgruppe, abspaltet, oder dass man

b) ein Halogenid der allgemeinen Formel

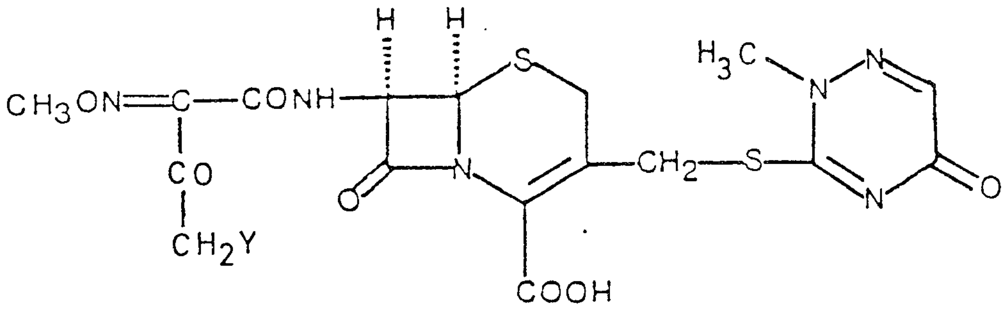

III

in der Y ein Halogenatom darstellt und die Carboxygruppe in geschützter Form vorliegen kann,

mit Thioharnstoff umsetzt, oder dass man

c) zur Herstellung eines leicht hydrolysierbaren Esters der Carbonsäure der Formel I diese einer entsprechenden Veresterung unterwirft, oder dass man

d) zur Herstellung von Salzen oder Hydraten der Verbindung der Formel I bzw. Hydraten dieser Salze die Verbindung der Formel I in ein Salz oder Hydrat bzw. ein Hydrat dieses Salzes überführt.

2. Verfahren nach Anspruch 1 zur Herstellung von (6R,7R)-7-[2-(2-Amino-4-thiazolyl)-2-[(Z)-methoxyimino]-acetamido]-3-[[(2,5-dihydro-2-methyl-5-oxo-as-triazin-3-yl)thio]methyl]-8-oxo-5-thia-1-azabicyclo[4.2.0]oct-2-en-2-carbonsäure sowie von Salzen dieser Verbindung und von Hydraten dieser Verbindung bzw. Salze, dadurch gekennzeichnet, dass man Alternative a), b) oder d) von Anspruch 1 durchläuft.

3. Verfahren nach Anspruch 1 zur Herstellung des Pivaloyloxymethylesters der in Anpruch 1 oder 2 definierten Carbonsäure, dadurch gekennzeichnet, dass man die Carbonsäure der Formel I einer entsprechenden Veresterung unterwirft.